(19) Europäisches Patentamt

European Patent Office

Office européen des brevets

(11) Veröffentlichungsnummer: **0 466 115 B1**

## (12) EUROPÄISCHE PATENTSCHRIFT

(45) Veröffentlichungstag der Patentschrift: **10.05.95**

(21) Anmeldenummer: **91111432.0**

(22) Anmeldetag: **09.07.91**

(51) Int. Cl.⁶: **C12P 17/00**, C12P 7/40,
//(C12P17/00,C12R1:38),
(C12P17/00,C12R1:74)

---

(54) **Mikrobiologische Oxidation von Ethylgruppen in Heterocyclen.**

---

(30) Priorität: **10.07.90 CH 2298/90**
**20.09.90 CH 3047/90**

(43) Veröffentlichungstag der Anmeldung:
**15.01.92 Patentblatt 92/03**

(45) Bekanntmachung des Hinweises auf die
Patenterteilung:
**10.05.95 Patentblatt 95/19**

(84) Benannte Vertragsstaaten:
**BE CH DE DK FR GB IT LI LU NL**

(56) Entgegenhaltungen:
EP-A- 0 274 146
EP-A- 0 348 901
DE-A- 3 822 595

**PATENT ABSTRACTS OF JAPAN**, unexamined applications, C field, Band 9, Nr. 260, 17.
Oktober 1985, The Patent Office Japanese
Govt.; Seite 151 C 309/

(73) Patentinhaber: **LONZA AG**
**(Geschäftsleitung: 4002 Basel)**
**CH-3945 Gampel/Wallis (CH)**

(72) Erfinder: **Kiener, Andreas, Dr.**
**Meisenweg 5**
**Visp,**
**Kanton Wallis (CH)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

---

**EP 0 466 115 B1**

**Beschreibung**

Die Erfindung betrifft ein neues mikrobiologisches Verfahren zur terminalen Oxidation von Ethylgruppen zur Carbonsäure.

Diese Carbonsäurederivate können beispielsweise als Zwischenprodukte für Pharmazeutika verwendet werden, wie beispielsweise Thiophen-2-essigsäure(2-Thienylessigsäure) zur Synthese von Penicillin-/Cephalosporin-Antibiotika (Ullmann, 1983, Band 23, S.219).

Eingehende Untersuchungen zur mikrobiologischen Oxidation von Alkylgruppen in aliphatischen Kohlenwasserstoffen wurden unter anderem mit dem Mikroorganismenstamm Pseudomonas oleovorans durchgeführt.

In Pseudomonas oleovorans ATCC 8062 und ATCC 29437 ist bekannt, dass die biochemische Oxidation von Alkanen in 3 Schritten zur entsprechenden Säure verläuft. Durch Einwirkung des Alkan-Hydroxylase-Komplexes entsteht zuerst der entsprechende Alkohol, der dann in 2 weiteren Schritten, katalysiert durch eine Alkohol-Dehydrogenase und eine Aldehyddehydrogenase,zur Säure überführt wird. In diesem Stamm liegen die Gene, welche für die Enzyme dieser Oxidation verantwortlich sind,auf dem Plasmid OCT (Witholt et al., TIBTECH, Vol.8, 1990, S.46-52).

Die mikrobiologische Oxidation von Alkylgruppen zu den entsprechenden Säuren durch Pseudomonas oleovorans ist bisher nur in Verbindungen mit linear gesättigten Alkylresten mit 6 bis 12 Kohlenstoffatomen und mit Ethylbenzol (Fukuda et al., Agric. Biol.Chem. 53 (12) S.3293-3299 (1989)) beschrieben.

Die Oxidation von Alkyl-substituierten cyclischen aromatischen oder gesättigten Kohlenwasserstoffen zur entsprechenden Carbonsäure mit Alkan-verwertenden Mikroorganismen,wie Rhodococcus, Mycobakterium und Pseudomonas,ist von Raymond, R.L., Process Biochemistry, 1969, S.71-74, beschrieben.

Nachteile dieses Verfahrens sind einmal, dass die Reaktion nicht spezifisch für Ethylgruppen ist, sondern auch Ringspaltungen eintreten können,und auch Methylgruppen in aromatischen Kohlenwasserstoffverbindungen oxidiert werden.

Die Aufgabe der vorliegenden Erfindung bestand darin,ein einfaches und einstufiges Verfahren zur mikrobiologischen Ethylgruppenoxidation in Heterocyclen zu entwickeln, mit dem die entsprechenden Säuren in guter Ausbeute und Reinheit isoliert werden können und der aromatische oder gesättigte Heterocyclus nicht gespalten wird.

Diese Aufgabe konnte auf überraschende Weise gemäss Anspruch 1 gelöst werden.

Als Substrate für die Umsetzung werden erfindungsgemäss 5- oder 6-Ring-Heterocyclen eingesetzt, die mit mindestens einer Ethylgruppe ringsubstituiert sind.

Zweckmässig weisen die genannten 5- oder 6-Ring-Heterocyclen ein Sauerstoff-, Stickstoff- oder Schwefelatom als Heteroatom auf.

Aus der Gruppe der 6-Ring-Heterocyclen werden bevorzugt solche mit Stickstoffheteroatom ausgewählt.

Geeignete Vertreter der 5-Ring-Heterocyclen sind die Thiophene, Furane, Pyrrole, Thiazole, die Pyrazole oder die Imidazole. Insbesondere werden als 5-Ring-Heterocyclen 2-Ethyl-thiophen oder 2-Ethyl-furan angewendet.

Geeignete Vertreter der 6-Ring-Heterocyclen sind die Pyridine, Pyrimidine, Pyrazine oder die Pyridazine. Insbesondere werden als 6-Ring-Heterocyclen 3-Ethyl-pyridin, 2-Ethylpyrazin oder 5-Ethyl-2-methyl-pyridin angewendet.

Zweckmässig werden die Enzyme der Alkan- und/oder Alkanol-verwertenden Mikroorganismen induziert. Der Begriff Alkane bzw. Alkanole umfasst ebenso substituierte Alkane bzw. Alkanole (alkylierte cyclische Verbindungen) wie beispielsweise Phenyl-substituiertes Ethan (Ethylbenzol).

Die Enzyminduktion kann sowohl mit Verbindungen durchgeführt werden, die dem Mikroorganismus als Kohlenstoff- und Energiequelle dienen, wie Alkane, Alkanole, alkylierte cyclische Verbindungen, wie beispielsweise Octan, Octanol, Dodecan, Dodecanol, Hexan, Hexanol, Ethylbenzol, als auch mit Verbindungen, die dem Mikroorganismus nicht als Kohlenstoff- und Energiequelle dienen, wie beispielsweise Dicyclopropylketon, Dicyclopropylmethanol oder Diethoxyethan, die bereits bei Grund et al., J.Bacteriol. 123, S.546-556, (1975) beschrieben sind.

Vorzugsweise wird die Enzyminduktion für Pseudomonas oleovorans mit n-Octan, für Candida tropicalis mit n-Hexadecan und für Rhodococcus rhodochrous mit n-Decan durchgeführt.

Ueblicherweise wird die Zufuhr der zur Induktion verwendeten Verbindungen während der Umsetzung des Substrats unterbunden. Die Umsetzung des Substrates kann jedoch auch in Anwesenheit des Enzyminduktors stattfinden.

Vorzugsweise wird die Zufuhr der zur Induktion verwendeten Verbindungen während der Umsetzung des Substrates entweder durch Stoppen der Zufuhr oder durch Abzentrifugieren der Zellen unterbunden.

2

Die Umsetzung kann mit Alkan- und/oder Alkanol-verwertenden Mikroorganismen, wie beispielsweise mit denen der Gattung Pseudomonas, mit Hefen der Gattung Candida und mit Mikroorganismen der Gattung Rhodococcus, durchgeführt werden.

Für das Verfahren ebenso geeignet sind Mutanten dieser Mikroorganismen sowie andere Mikroorganismen, in die entweder durch Konjugation oder durch gentechnologische Methoden die für die Umsetzung notwendige genetische Information eingebracht wurde, und damit die wirksamen Enzyme für die Umsetzung bilden.

Als Mikroorganismen können die Alkan-verwertenden Mikroorganismenstämme Pseudomonas oleovorans mit der Bezeichnung ATCC 8062 oder ATCC 29347, Hefen der Spezies Candida tropicalis mit der Bezeichnung ATCC 32113 und Rhodococcus rhodochrous mit der Bezeichnung ATCC 19067 angewendet werden, vorzugsweise Pseudomonas oleovorans mit der Bezeichnung ATCC 29347. Die Mikroorganismenstämme Pseudomonas oleovorans ATCC 8062 oder ATCC 29347 und Rhodococcus rhodochrous ATCC 19067 sowie Hefen der Gattung Candida tropicalis ATCC 32113 sind bei der American Type Culture Collection, 12301 Parklawn Drive, Rockville, Maryland 20852, USA, hinterlegt worden.

Die genannten Stämme wachsen mit Alkanen, Alkanolen oder mit alkylierten cyclischen Verbindungen in einem Mineralsalzmedium (Kulla et al., Arch. Microbiol 135, 1983, S.1-7) oder in einem Komplexmedium ("Nutrient Broth Nr.2", Oxoid Ltd., England).

Vor der Substratzugabe werden die Zellen auf übliche Weise gezüchtet, und anschliessend wird die Umsetzung des Substrats bei einer optischen Dichte von 1 bis 200 bei 650 nm im Kulturmedium durchgeführt, vorzugsweise bei einer optischen Dichte von 5 bis 100 bei 650 nm.

Die Umsetzung kann entweder unter einmaliger oder kontinuierlicher Substratzugabe erfolgen, so dass die Substratkonzentration im Kulturmedium 20% (w/v) nicht übersteigt, wobei (w/v) eine Abkürzung für Gewicht pro Volumen bedeutet.

Vorzugsweise erfolgt die Substratzugabe so, dass die Substratkonzentration im Kulturmedium 5% (w/v) nicht, insbesondere dass die Substratkonzentration im Kulturmedium nicht 1% (w/v) übersteigt.

Die Umsetzung wird zweckmässig in einem pH-Bereich von 4 bis 11, vorzugsweise von 6 bis 10, durchgeführt.

Die Umsetzung wird üblicherweise bei einer Temperatur von 15 bis 50°C, vorzugsweise bei einer Temperatur von 25 bis 40°C, durchgeführt.

Die Umsetzung wird zweckmässig in einer Zeit von 1 Stunde bis zu mehreren Tagen durchgeführt, vorzugsweise wird die Umsetzung mit einem kontinuierlichen Verfahren über mehrere Tage durchgeführt.

Nach der Umsetzung können die entsprechenden Säuren auf bekannte Art und Weise isoliert werden.

Beispiel 1

(2-Methyl-pyridin-5-essigsäure)

Pseudomonas oleovorans ATCC 29347 wurde in einem Mineralsalzmedium (Kulla et al., Arch.Microbiol. 135, 1983, S.1-7) mit n-Octan als einziger Kohlenstoff- und Energiequelle bei 30°C und bei einem pH-Wert von 7 angezogen.

Anschliessend wurden die Zellen 2 mal mit dem gleichen Mineralsalzmedium gewaschen und eine optische Dichte von 10 bei 650 nm in 100 ml Mineralsalzmedium eingestellt. Zu dieser Zellsuspension wurde 1 mMol 5-Ethyl-2-methyl-pyridin hinzugegeben, was einer Substratkonzentration von 0,12% (w/v) entspricht. Nach einer Inkubation von 16 Stunden bei 30°C wurde in Abwesenheit von n-Octan 0,5 mMol 2-Methyl-pyridin-5-essigsäure, entsprechend einer Ausbeute von 50%, bezogen auf eingesetztes Ethyl-methyl-pyridin, erhalten. Unter diesen Bedingungen konnte keine Oxidation der Methylgruppe in Ethyl-methyl-pyridin nachgewiesen werden.

Die Beispiele 2 bis 5 wurden entsprechend zu Beispiel 1 mit einer Menge von 1 mMol Substrat pro 100 ml Zellsuspension durchgeführt und sind in Tabelle 1 zusammengefasst.

Tabelle 1:

| Beispiel | | Konzentration des Substrates in % (w/v) im Kulturmedium | Reaktionszeit in h | Endprodukt | Ausbeute in % (w/v) |
|---|---|---|---|---|---|
| 2 | 3-Ethyl-pyridin | 0,108 | 16 | Pyridin-3-essigsäure | 20 |
| 3 | 2-Ethyl-pyrazin | 0,109 | 16 | Pyrazin-2-essigsäure | 20 |
| 4 | 2-Ethyl-thiophen | 0,113 | 16 | Thiophen-2-essigsäure | 20 |
| 5 | 2-Ethyl-furan | 0,097 | 16 | Furan-2-essigsäure | 20 |

**Patentansprüche**

1. Mikrobiologisches Verfahren zur terminalen Oxidation von Ethylgruppen zur Carbonsäure, dadurch gekennzeichnet, dass man als Substrat einen 5- oder 6-Ring Heterocyclus, substituiert mit mindestens einer Ethylgruppe, mittels Alkan- und/oder Alkanol-verwertenden Mikroorganismen zur entsprechenden Carbonsäure umsetzt, wobei die resultierende Carbonsäure nicht weiter katabolisiert wird.

2. Verfahren nach Patentanspruch 1, dadurch gekennzeichnet, dass die Enzyme des Mikroorganismus entweder mit Verbindungen induziert werden, die dem Mikroorganismus als Kohlenstoff- und Energiequelle dienen, oder mit Verbindungen, die dem Mikroorganismus nicht als Kohlenstoff- und Energiequelle dienen.

3. Verfahren nach Patentanspruch 1 oder 2, dadurch gekennzeichnet, dass die Umsetzung mit den Alkan-verwertenden Mikroorganismenstämmen Pseudomonas oleovorans mit der Bezeichnung ATCC 8062 oder ATCC 29347 oder wirksamen Mutanten von diesen Stämmen durchgeführt wird.

4. Verfahren nach Patentanspruch 1 oder 2, dadurch gekennzeichnet, dass man die Umsetzung mit Alkan-verwertenden Mikroorganismenstämmen,wie Hefen der Spezies Candida tropicalis mit der Bezeichnung ATCC 32113 oder einer wirksamen Mutante von diesen, durchführt.

5. Verfahren nach Patentanspruch 1 oder 2, dadurch gekennzeichnet, dass man die Umsetzung mit dem Alkan-verwertenden Mikroorganismenstamm Rhodococcus rhodochrous mit der Bezeichnung ATCC 19067 oder einer wirksamen Mutante von diesem durchführt.

6. Verfahren nach einem oder mehreren der Patentansprüche 1 bis 5, dadurch gekennzeichnet, dass die Umsetzung entweder unter einmaliger oder unter kontinuierlicher Substratzugabe durchgeführt wird, so dass die Substratkonzentration im Kulturmedium 20% (w/v) nicht übersteigt.

7. Verfahren nach einem oder mehreren der Patentansprüche 1 bis 6, dadurch gekennzeichnet, dass die Umsetzung bei einem pH-Wert von 4 bis 11 durchgeführt wird.

8. Verfahren nach einem oder mehreren der Patentansprüche 1 bis 7, dadurch gekennzeichnet, dass man die Umsetzung bei einer Temperatur von 15 bis 50 °C durchführt.

9. Verfahren nach einem der Patentansprüche 1 bis 8, dadurch gekennzeichnet, dass man als Substrat einen aromatischen 5- oder 6-Ring-Heterocyclus, substituiert mit einer Ethylgruppe, einsetzt, der ein oder mehrere Heteroatome aus der Reihe Sauerstoff, Stickstoff oder Schwefel enthält.

## Claims

1. Microbiological process for the terminal oxidation of ethyl groups to the carboxylic acid, characterised in that as the substrate a 5 or 6-membered heterocycle, substituted with at least one ethyl group, is reacted with the aid of alkane and/or alkanol-consuming microorganisms to the respective carboxylic acid, the resulting carboxylic acid not being further catabolized.

2. Process according to claim 1, characterised in that the enzymes of the microorganism are induced either with compounds that are used by the microorganism as a carbon and energy source or with compounds which are not used by the microorganism as a carbon and energy source.

3. Process according to claim 1 or 2, characterised in that the reaction is carried out with the alkane-consuming microorganism strains Pseudomonas oleovorans denoted ATCC 8062 or ATCC 29347 or effective mutants of these strains.

4. Process according to claim 1 or 2, characterised in that the reaction is carried out with alkane-consuming microorganism strains like yeasts of the species Candida tropicalis denoted ATCC 32113 or an effective mutant thereof.

5. Process according to claim 1 or 2, characterised in that the reaction is carried out with the alkane-consuming microorganism strain Rhodococcus rhodochrous denoted ATCC 19067 or an effective mutant thereof.

6. Process according to one or more of claims 1 to 5, characterised in that the reaction is carried out either with a single or continuous addition of the substrate so that the concentration of the substrate in the culture medium does not exceed 20 % (w/v).

7. Process according to one or more of claims 1 to 6, characterised in that the reaction is carried out at a pH value of from 4 to 11.

8. Process according to one or more of claims 1 to 7, characterised in that the reaction is carried out at a temperature of from 15 to 50 °C.

9. Process according to one or more of claims 1 to 8, characterised in that as the substrate an aromatic 5 or 6-membered heterocycle, substituted with one ethyl group, is employed, said heterocycle containing one or more heteroatoms from the group of oxygen, nitrogen or sulfur.

**Revendications**

1. Procédé microbiologique pour l'oxydation terminale de groupes éthyle en acide carboxylique, caractérisé en ce que l'on met en réaction en tant que substrat un hétérocycle à 5 ou 6 sommets, substitué par au moins un groupe éthyle au moyen de micro-organismes utilisant l'alcane et/ou l'alcanol pour l'obtention de l'acide carboxylique correspondant, l'acide carboxylique résultant n'étant pas catabolisé ultérieurement.

2. Procédé selon la revendication 1, caractérisé en ce que les enzymes du micro-organisme sont induites soit par des composés qui servent au micro-organisme en tant que source de carbone et d'énergie, soit par des composés qui ne servent pas de source de carbone et d'énergie au micro-organisme.

3. Procédé selon la revendication 1 ou 2, caractérisé en ce que la réaction est conduite avec les souches de micro-organismes utilisant l'alcane Pseudomonas oleovorans portant la désignation ATCC 8062 ou ATCC 29347 ou les mutants efficaces de ces souches.

4. Procédé selon la revendication 1 ou 2, caractérisé en ce que l'on conduit la réaction avec des souches de micro-organismes utilisant l'alcane, comme par exemple des levures de l'espèce Candida tropicalis portant la désignation ATCC 32113 ou un mutant efficace de celle-ci.

5. Procédé selon la revendication 1 ou 2, caractérisé en ce que l'on effectue la réaction avec la souche de micro-organisme utilisant l'alcane, Rhodococcus Rhodochrous portant la désignation ATCC 19067 ou un mutant efficace de cette souche.

6. Procédé selon une ou plusieurs des revendications 1 à 5, caractérisé en ce que la réaction est conduite soit par addition en une seule fois soit par addition continue du substrat afin que la concentration du substrat dans le milieu de culture ne dépasse pas 20 % (pds/v).

7. Procédé selon une ou plusieurs des revendications 1 à 6, caractérisé en ce que la réaction s'effectue à une valeur de pH de 4 à 11.

8. Procédé selon une ou plusieurs des revendications 1 à 7, caractérisé en ce que l'on conduit la réaction à une température de 15 à 50 °C.

9. Procédé selon l'une des revendications 1 à 8, caractérisé en ce qu'en tant que substrat, on met en oeuvre un hétérocycle aromatique à 5 ou 6 sommets, substitué par un groupe éthyle qui contient 1 ou plusieurs hétéroatomes de la série oxygène, azote ou soufre.